# FASCICULE DE BREVET EUROPEEN

(11) **EP 0 456 580 B1**
(45) Date de publication et mention de la délivrance du brevet: **05.03.1997**
(21) Numéro de dépôt: 91401214.1
(22) Date de dépôt: 07.05.1991
(51) Int. Cl.: A61F 2/34, A61F 2/32

(54) **Prothèse de la hanche**
Hüftgelenkprothese
Hip joint prosthesis

(30) Priorité: 10.05.1990 LU 87732
(43) Date de publication de la demande: 13.11.1991
(73) Titulaire: BCM HOLDING S.A., L-1724 Luxembourg (LU)
(72) Inventeur: Karaqui, Najwa, Abidjan (CI)
(74) Mandataire: Wagret, Frédéric

(56) Documents cités:
- EP-A- 0 214 885
- EP-A- 0 237 751
- FR-A- 2 537 868
- FR-A- 2 598 609
- US-A- 4 662 891
- US-A- 4 795 469

## Description

La présente invention concerne une prothèse totale de la hanche comportant, de façon conventionnelle, d'une part un implant cotyloïdien apte à être vissé sur le support osseux de la hanche et une tige fémorale implantée au sommet du fémur et se terminant par une rotule elle-même introduite dans le logement complémentaire et récepteur prévu sur l'implant cotyloïdien.

Les prothèses de ce type comportent donc deux éléments destinés à assurer l'articulation rotoïdale du fémur sur la hanche et constitué par conséquent de l'implant fixe (cotyloïdien) définissant une capacité ou un logement récepteur, et d'autre part l'implant fémoral se terminant par la rotule destiné à être mis en place et retenu dans son logement.

On connaît depuis longtemps des prothèses de ce type qui, pour donner complètement satisfaction, doivent répondre à de nombreux objectifs, lesquels sont loin d'être réunis cumulativement dans les diverses formes de réalisation actuellement connues.

En premier lieu, l'implant cotyloïdien fixe doit bénéficier d'un ancrage stable sur son support, évitant par conséquent tout mouvement relatif de l'implant par rapport à son siège osseux récepteur.

Et cette stabilité implique que non seulement on évite tout jeu relatif entre l'implant et le siège osseux, mais que également l'implant, une fois en place, ne subisse pas de déplacement continu parasite par rapport à son support. Tel est le cas notamment de certains implants cotyloïdiens connus, tels que montrés dans le document US.4.795.469 ou US.4.662.891, qui sont mis en place par une opération de vissage, l'implant de forme générale hémisphérique comportant sur son pourtour des filets hélicoïdaux permettant par conséquent un effet de vissage dans la masse osseuse ; il arrive que le phénomène de rotation et d'auto-taraudage dû à la présence des filets hélicoïdaux sur l'implant, provoque, par suite des mouvements liés à la marche du sujet, un phénomène de taraudage continu provoquant par conséquent l'avancement de l'implant cotyloïdien dans son support jusqu'à traverser l'os du bassin.

Il est donc nécessaire que l'implant cotyloïdien assure par sa morphologie un équilibre et une répartition des forces et des pressions s'exerçant entre le fémur et la hanche et telles que les forces et pressions s'équilibrent sans induire de composantes parasites susceptibles d'entraîner par la répétition des contraintes locales et ponctuelles un déplacement de l'implant par rapport à son berceau récepteur.

Il est également apparu souhaitable de prévoir une possibilité de retrait de la prothèse et notamment par conséquent d'extraction de l'implant fixe cotyloïdien après une période de temps de présence ayant pu donner naissance à des effets secondaires nocifs nécessitant le remplacement de l'implant ; et si 'implant se trouve noyé et incrusté dans la masse osseuse, son extraction peut présenter des difficultés et entraîner des dommages au niveau du support ; il est donc souhaitable que la configuration de l'implant permette d'effectuer son extraction par auto-taraudage rétroactif, ou rétro-autotaraudage en évitant par conséquent une agression du tissu osseux lors d'une extraction éventuelle.

Il est encore nécessaire que la prothèse puisse être mise en place de façon parfaitement ajustée sur son siège support et que le praticien puisse en conséquence surveiller et repérer visuellement en permanence la positionnement instantané de l'implant en cours de vissage par rapport à la paroi réceptrice.

Enfin, il est encore nécessaire pour assurer une cohabitation et une symbiose de l'implant dans sa niche réceptrice, d'éviter une fragilisation des fractions osseuses notamment au niveau compris entre deux ailettes ou filets ; et plus particulièrement il convient que la configuration de l'implant évite tout tassement et à plus forte raison tout écrasement du tissu osseux sous la forme lamellaire insérée entre deux filets.

Les dispositifs actuellement connus ne remplissent qu'incomplètement et imparfaitement ces objectifs et surtout ils se révèlent impropres à les assurer tous cumulativement.

La prothèse fémorale selon la présente invention permet d'atteindre ces divers objets et permet la mise en place de la prothèse par une manoeuvre opératoire simple faisant face aux différents types morphologiques de l'articulation de la hanche tout en évitant le recours à un matériel auxiliaire complexe.

Notamment l'implant fixe cotyloïdien, tout en utilisant un système de vissage dont le principe est connu en soi, permet un ancrage stable respectant les données de l'anatomie et en évitant une résection osseuse trop importante.

Un autre objet de l'invention est de réaliser un implant permettant par un effet d'auto-rétrotaraudage, le retrait de la prothèse sans détérioration du tissu porteur lors de cette opération.

La prothèse conforme à l'invention assure par ailleurs une répartition et un équilibre entre les forces antagonistes s'exerçant entre le massif osseux du bassin et le membre inférieur.

A cet effet, l'invention concerne une prothèse totale de la hanche comportant un implant cotyloïdien constitué d'une coquille hémisphérique comportant sur sa paroi extérieure, dans la partie proche de l'équateur, un filetage hélicoïdal et spiralé laissant dégagée au sommet de la demi-sphère une calotte supérieure, le filetage présentant des propriétés d'auto-taraudage permettant ainsi une attaque du milieu d'implantation pour l'insertion et le passage des filets dans le sens de l'implantation de la prothèse, les filets étant interrompus à intervalles réguliers par des gorges disposées selon un axe médian et définissant des segments de filets ou ailettes répartis en hélice spiralée sur la paroi de la coquille hémisphérique, au moins dans la zone proche de l'équateur,
caractérisée en ce que le filetage présente des propriétés de rétro-autotaraudage pour une attaque du milieu d'implantation et le passage de filets dans le sens du retrait de la prothèse.

De préférence et selon une forme de réalisation plus particulière, les bords de chaque gorge, définis par les bords transversaux et terminaux des segments de filets superposés en hélice, sont parallèles à l'axe médian de la gorge, lui-même disposé radialement, de sorte que les deux bords de deux segments de filets voisins, délimitant entre eux une gorge séparant ces deux segments successifs le long de l'hélice, sont parallèles entre eux et non convergents, et ainsi chaque bord transversal et terminal d'un segment de filets constitue un tranchant d'attaque apte à aborder la matière taraudée selon un angle inférieur à un droit et tel que la matière taraudée reçoit en premier lieu l'attaque de la pointe extérieure dudit bord transversal et terminal du segment de filet pénétrant dans ladite matière taraudée.

Selon encore une autre caractéristique importante de l'invention, chaque gorge comporte une largeur constante, comprise entre 2 et 5 millimètres, et son fond épouse la forme d'un segment de sphère prolongeant la calotte sphérique supérieure.

Selon une autre caractéristique, les segments de filets ou ailettes définissant ensemble le filetage d'auto-taraudage, sont répartis selon une hélice spiralée de pas compris entre 3 et 4 millimètres et de préférence voisin de 3,5 millimètres.

Selon encore une autre caractéristique de l'invention, le bord de chaque gorge, défini par le segment d'attaque du filet d'auto-taraudage est parallèle à l'axe médian radial des gorges n'est pas convergent, et forme ainsi un angle « α » avec le rayon correspondant passant par la pointe extérieure périphérique du filet, de sorte que le filet attaque la matière osseuse par ladite pointe en premier lieu.

D'autres caractéristiques et avantages de l'invention ressortiront de la description qui suit et qui est donnée en rapport avec une forme particulière de réalisation présentée à titre d'exemple non limitatif et en se référant aux dessins annexés.

La Figure 1 représente une vue en perspective de la partie extérieure (convexe) de la coquille hémisphérique ou support constituant l'implant cotyloïdien.

La Figure 2 représente une vue en élévation latérale de cette même coquille support.

La Figure 3 représente une vue de détail montrant la configuration des gorges séparant deux rangées d'ailettes ou segments de filets superposées sur la paroi extérieure de la coquille précédente.

La Figure 4 représente une vue en coupe horizontale montrant en position supérieure la coquille hémisphérique constituant l'implant cotyloïdien et en position inférieure le noyau destiné à être engagé à l'intérieur de cette coquille pour contenir la rotule.

La Figure 5 montre une vue en coupe verticale d'un détail révélant la configuration des ailettes ou segments de filets montés sur le pourtour de la cupule définissant l'implant cotyloïdien.

La Figure 6 montre une vue de détail visant à présenter la configuration du départ d'une ailette ou segment de filet (ou sa fin) au niveau d'une gorge.

La Figure 7 montre une vue en plan par dessous de la coquille nue et sans le noyau.

La Figure 8 montre l'outil de manoeuvre de la coquille en vue de son implantation d'une part et en vue de son extraction d'autre part.

La Figure 9 montre une vue en élévation frontale (vis-à-vis du sujet récepteur) de la tige constituant l'implant fémoral.

La Figure 10 montre une vue de cette même tige comportant la tête.

La Figure 11 montre une section de la tige au niveau de l'embase selon la ligne XI - XI de la Figure 9.

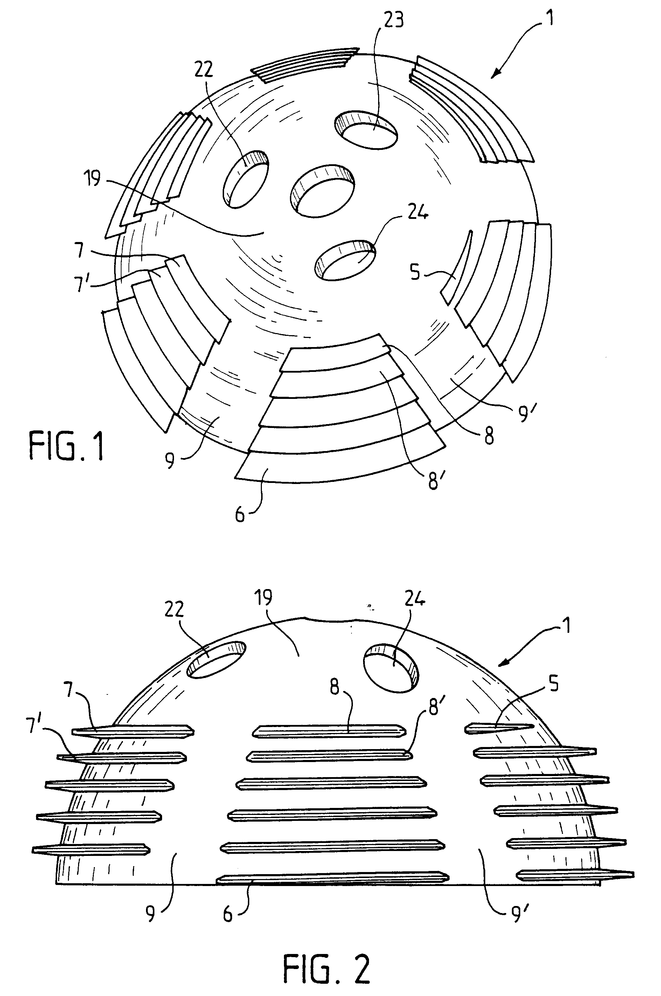

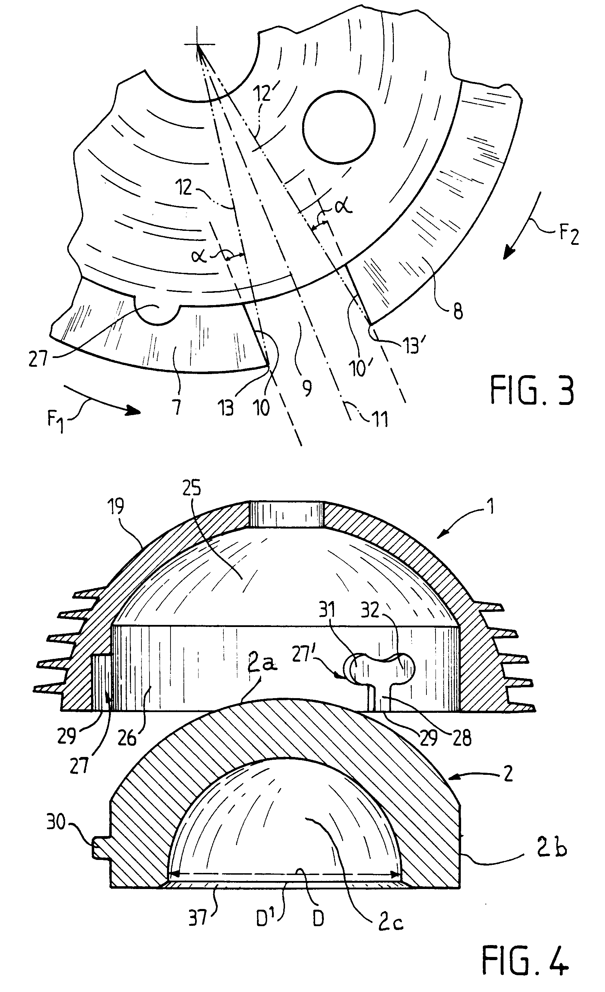

On voit selon l'ensemble des Figures que la prothèse se compose, de façon conventionnelle, d'un implant cotyloïdien destiné à être mis en place sur le bassin osseux de la hanche et constitué d'une coquille ou cupule support représentée notamment en perspective par sa face convexe extérieure à la Figure 1 et représentée en coupe à la Figure 4.

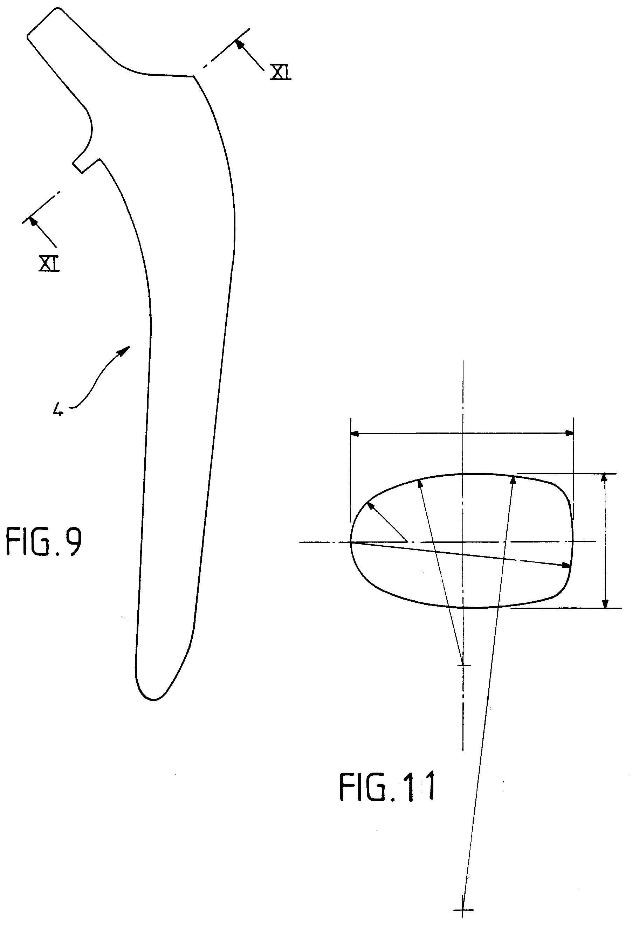

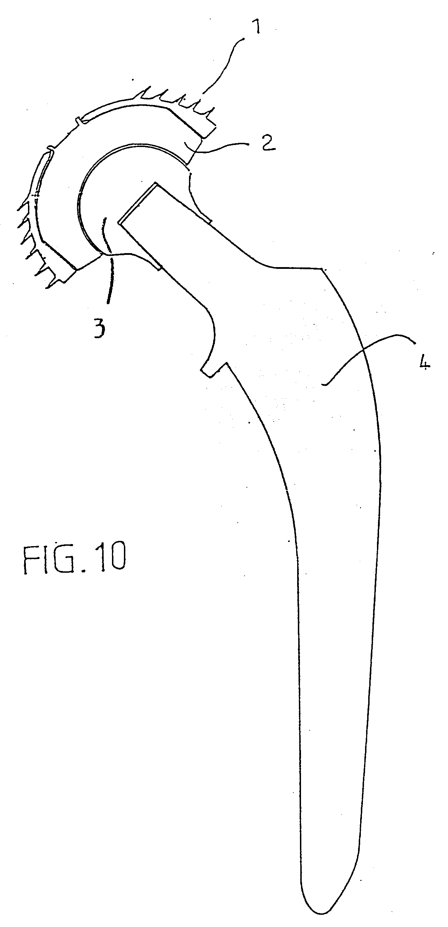

A l'intérieur de cette coquille 1 vient se positionner un noyau intermédiaire 2 représenté particulièrement à la Figure 4 et qui contient un logement sphéroïdal concave récepteur de la rotule 3 prolongeant à son sommet la tige 4 représentée notamment à la Figure 9 et à la Figure 10.

On décrira successivement l'implant fixe ou implant cotyloïdien puis la tige fémorale.

L'implant cotyloïdien selon les Figures 1, 2, 3 et 4 répond à divers impératifs.

Un premier objet de cet implant est d'offrir un champ d'indications plus large à l'utilisation d'un système de vissage comme solution d'ancrage.

Selon un second objectif l'implant cotyloïdien rend cette solution satisfaisante en respectant les données de l'anatomie tout en limitant la résection osseuse.

Enfin, il permet une manoeuvre opératoire simple adaptée aux différents types morphologiques de l'articulation de la hanche tout en évitant le recours à un matériel auxiliaire complexe lors de la mise en place.

L'implant fixe est constitué d'une coquille métallique 1 en forme de cupule sensiblement hémisphérique dont la paroi externe convexe assure un ancrage dans le support osseux grâce à un filetage d'une part auto-taraudeur lors de la mise en place mais également auto-rétrotaraudeur lors du retrait éventuel ultérieur.

Aux fins d'assurer l'implantation et l'ancrage initial, la coquille hémisphérique constituant l'implant fixe comporte un système de filetage qui sera décrit ci-après.

Selon les Figures 1, 2 et 5, on voit que le filetage est constitué d'une succession de filets disposés selon une hélice spiralée depuis le sommet 5 jusqu'au filet terminal 6.

Et ces filets successifs sont répartis selon des segments de filets superposés 7, 7', 8, 8' qui définissent entre eux des gorges 9, 9' disposées radialement sur le pourtour de la paroi convexe hémisphérique de la coquille.

Selon une première caractéristique importante de l'invention et qui est illustrée à la Figure 3, les bords transversaux et terminaux de chaque segment de filet 8 et 7 sont disposés de façon parallèle entre eux et définissent entre ces deux bords respectivement 10 et 10' la gorge 9 elle-même positionnée selon un axe 11 radial par rapport au volume hémisphérique de la coquille.

On voit selon la Figure 3 que les deux bords transversaux des segments de filets 7 et 8, respectivement 10 et 10', sont parallèles entre eux et parallèles à l'axe médian radial 11 de la gorge 9.

On voit également que dans ces conditions chacun des bords 10 et 10' forme avec le rayon défini par la droite 12, 12' joignant la pointe 13, 13' du segment de filet 7 ou 8 au sommet polaire de la sphère, un angle α différent du droit.

Dans ces conditions on comprend que lorsque la coquille est engagée en rotation selon la flèche F1 (Figure 3) la pointe 13 attaque initialement la matière osseuse soumise au taraudage avant l'ensemble du bord 10 en permettant ainsi une attaque régulière et une pénétration parfaite du filet dans la matière porteuse.

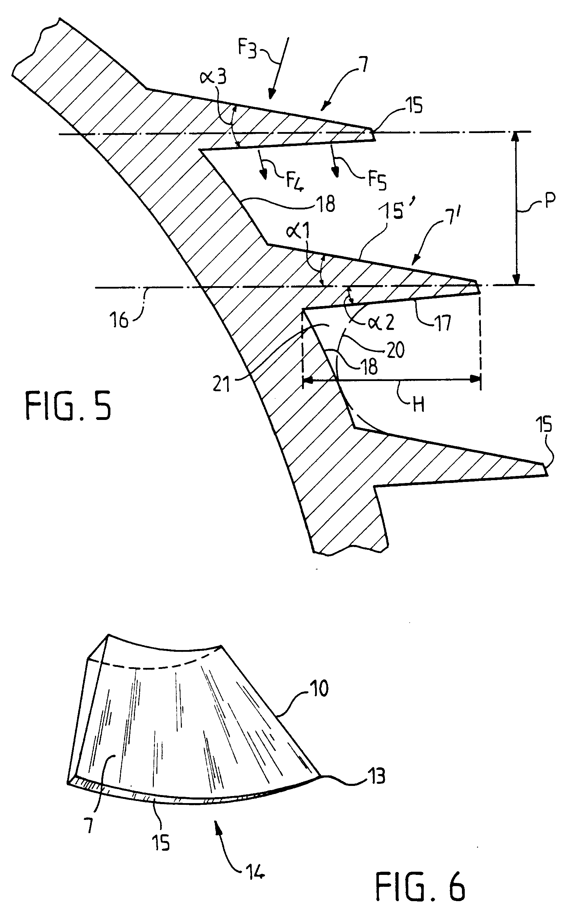

La Figure 6 montre d'ailleurs de façon plus détaillée la configuration dans l'espace du filet 7 au niveau de son bord d'attaque d'auto-taraudage 10.

On voit que si le filet présente une certaine épaisseur, au niveau du bord d'attaque 10 il se termine selon un fil coupant en permettant par conséquent l'effet de sectionnement et de pénétration dans la masse du support, tandis que l'angle 13 représente la pointe ou poinçon d'attaque initiale du filet en cours de pénétration par auto-taraudage ; sur son bord curviforme extérieur 14 le filet comporte, au delà de la pointe 13, un biseautage 15 qui est notamment représenté en coupe à la Figure 5 et qui émousse ainsi le tranchant de ce bord extérieur.

Et selon une autre caractéristique et un autre avantage de l'invention, le bord 10' situé en regard du bord 10, et terminant le filet ou segment de filet voisin 8, est conformé de façon symétrique par rapport au bord 10, de sorte la rotation de l'implant dans un sens inverse au précédent, c'est-à-dire selon la flèche F2, inverse de la flèche F1, et correspondant à la rotation de l'ensemble lors du retrait, provoque le même phénomène d'attaque de la matière par la pointe 13'.

On permet ainsi à la coquille support d'opérer une action d'auto-taraudage (par le bord 10 et la pointe 13 lors de la mise en place) mais symétriquement une action d'auto-rétrotaraudage (par la pointe 13' et le fil du bord 10') lors de l'opération de retrait éventuel.

On évite ainsi lors d'un retrait ultérieur de l'implant une agression de la matière osseuse reconstituée ou implantée dans les gorges radiales 9, 9' etc...

Selon une autre caractéristique de l'invention, la conformation des filets ou segments de filets en coupe, et illustrée à la Figure 5, permet une grande stabilité primaire de l'implant après sa mise en place grâce à une répartition et à un report des forces s'exerçant au niveau de chaque segment de filet inséré dans la matière osseuse vivante.

Chaque segment de filet 7, 7', 7'' correspond en effet à une configuration en coupe représentée à la Figure 5 et définie par les angles respectivement α1 et α2 formés d'une part entre la droite définie par la coupe radiale de la face supérieure 15' du filet par rapport à un plan horizontal médian 16 et d'autre part entre la droite définie par la coupe radiale de la face inférieure 17 du filet 7, 7', 7'' par rapport au même plan médian 16.

Et on voit notamment pour le filet 7' que l'angle α1 (formé entre la droite coupant radialement le plan supérieur 15' du filet 7' et le plan médian 16) est supérieur à l'angle α2 (formé entre ce même plan médian 16 et la droite correspondant à la coupe radiale du plan inférieur 17 du filet 7').

Dans ces conditions les forces convergentes qui s'exercent selon une direction normale à la face supérieure 15' du filet 7, 7', 7'' selon la flèche F3, sont reportées par le segment de filet 7, 7', 7'' selon une direction également normale par rapport à la face inférieure 17 du filet conformément aux flèches F4 et F5.

Et l'on voit que dans ces conditions les forces convergentes et centripètes qui s'exercent sur la paroi supérieure du filet sont reportées depuis la paroi inférieure du filet vers la matière osseuse dans des directions divergentes et centrifuges.

De plus et selon la Figure 5, les filets répartis sur 5 à 6 tours d'hélice selon les tailles, correspondent aux caractéristiques préférentielles dimensionnelles ci-après.

La hauteur du filet (référence H sur la Figure 5) est voisin de 3 mm. ; le pas du filet (référence P sur la même Figure) est voisin de 3,5 mm.

L'angle α1 est de l'ordre de 10° tandis que l'angle α2 est de l'ordre de 3°,

L'angle au sommet α3 (correspondant à la somme des angles α1 et α2) est ainsi de l'ordre de 13°.

Enfin, on note selon la Figure 5 que le fond du filet correspondant à la paroi 18 est contenu dans une couronne sphérique qui prolonge la calotte sphérique supérieure 19 et le fond est confondu avec la paroi de la coquille hémisphérique contenant donc non seulement la calotte 19 mais les parois des gorges 9, 9' et tous les fonds de filet.

A cet effet, la paroi 18 constituant le fond des filets est usinée pour reconstituer au niveau de chaque fond de filet une paroi extérieure de profil sphérique prolongeant les parois hémisphériques de la coquille et donc de même rayon.

Selon cette caractéristique on évite d'une part une fragilisation de la matière osseuse par suite de la compression qui s'opèrerait en cet endroit si le fond de filet n'était pas proprement dégagé comme décrit précédemment ; on voit en effet selon la Figure 5 entre les filets 7' et 7'' une ligne curviforme en pointillés 20 qui illustre un fond de filet conventionnel.

Et on voit que la présence d'un filet dont le fond suivrait la ligne 20 aboutirait à comprimer la matière osseuse dans le fond de filet, cette matière ne pouvant pas occuper l'espace situé dans l'angle inférieur 21, d'où agression du tissu vivant source de nécrose ou sclérose.

Au contraire, dans le cadre de la présente invention, la conformation des fonds de filets prolongeant la paroi extérieure hémisphérique de la coquille dégage cet angle 21 et permet par conséquent à la matière osseuse d'occuper cette zone sans être modifiée dans sa structure et sans subir de compression agressive lors de la mise en place.

De plus on évite ainsi de créer des zones de rupture dans la diminution du gradient des pressions exercées sur l'implant depuis la zone polaire jusqu'à la zone équatoriale ; les pressions s'exerçant ainsi depuis le haut jusqu'en bas de la coquille de façon continûment et régulièrement dégressive sans reconstituer une répartition des pressions en marche d'escalier.

Les caractéristiques de la coquille et des segments de filets de taraudage qui ont été décrites permettent ainsi une mise en place aisée grâce au mordant du filet avec possibilité d'extraction ultérieure non agressive.

L'implant cotyloïdien ainsi décrit présente une stabilité primaire et secondaire élevée.

Ceci d'une part du fait de l'importante surface de contact obtenue entre la partie prothétique et l'os (hauteur du filet et longueur du filetage).

Et d'autre part, du fait d'une configuration particulière du filetage dont la symétrie donne un aspect faussement rétentif permettant la conversion du champ des forces concentriques et centripètes en un gradient de pressions centrifuges transmises par le bord inférieur des segments de filets.

La coquille hémisphérique présente dans sa zone polaire un perçage de fenêtres sphériques, notamment au nombre de 3, 22, 23, 24 qui permettent, lors de la mise en place, une perception visuelle du fond du cotyle et une vérification constante par le praticien du positionnement de l'implant par rapport à sa niche osseuse réceptrice, autorisant donc un repérage instantané dans la pénétration de l'implant du moment où le contact "à quai" entre l'implant et son support est établi.

La Figure 4 représente une vue en coupe et perspective partielle de la capacité interne de la coquille 1 et du noyau 2 qu'elle contient.

Tandis que la coquille est réalisée en alliage métallique, le noyau est usiné dans une masse de polyéthylène à haute densité.

La capacité interne de la coquille cotyloïdienne 1 présente une concavité hémisphérique dans sa partie supérieure 25 tandis que sa baser à proximité du plan de l'équateur, adopte une configuration cylindrique 26.

Cette zone cylindrique reçoit intérieurement 3 gorges d'ancrage réparties de façon équidistante sur la circonférence de la paroi 26, et chacune de ces gorges 27, 27', est prévue avec une configuration générale en forme de trèfle ou de "T". L'âme centrale 28 de cette gorge débouche sur le bord inférieur de la coquille hémisphérique au niveau de son plan sensiblement équatorial par l'entrée 29. Cette entrée permettra l'engagement d'un plot ou ergot 30 venu radialement du noyau 2 ; la gorge 27 ou 27' s'étend depuis son débouché 29 dans une direction orthogonale par rapport au plan équatorial et elle se prolonge par les deux ailes latérales du "T" en conformant les deux logements curviformes 31 et 32 aptes à recevoir et à immobiliser l'ergot 30.

La disposition des deux logements symétriques 31 et 32 permettra à l'ensemble d'être opérationnel pour le mouvement d'implantation ou de retrait de la prothèse cotyloïdienne.

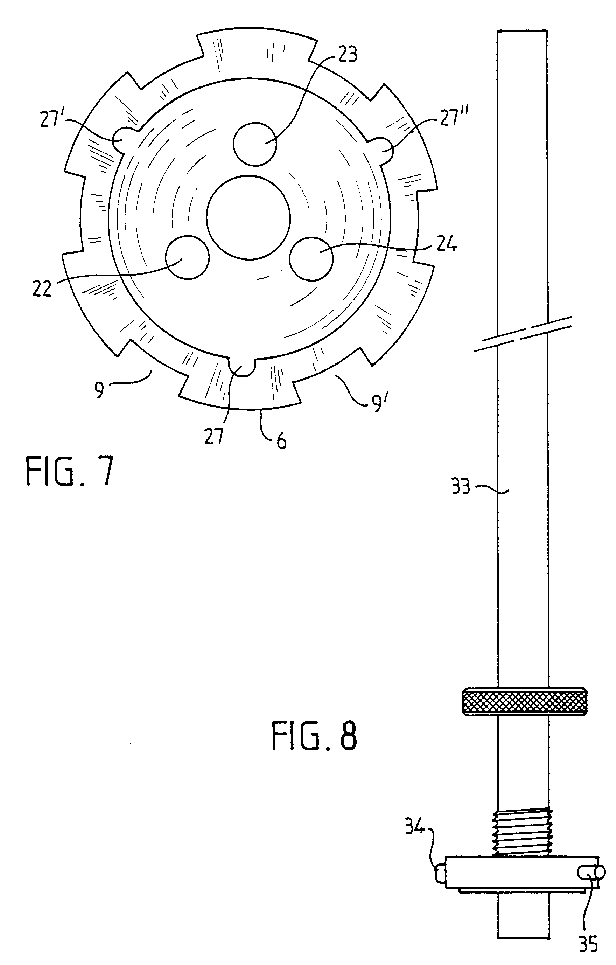

En effet, chaque gorge 27, 27' est prévue pour recevoir l'engagement de l'outil de mise en place représenté à la Figure 8.

A la base du manche 33 la couronne 34 comporte également les ergots 35, qui pourront être ainsi engagés dans les trois gorges correspondantes (dont sont représentées les deux gorges 27, 27') et venir en position de blocage soit dans un logement 31, soit dans un logement 32, suivant le sens de rotation de l'implant, c'est-à-dire suivant que l'opération se fait en vue de l'implantation (mise en place) ou en vue du retrait.

L'ensemble ainsi décrit permet donc, en coopérant avec la conformation des ailettes ou segments de filets auto-taraudeurs et auto-rétrotaraudeurs, une fonction bivalente du système susceptible d'être manoeuvré également avec la même facilité tant pour les opérations de mise en place et d'implantation que pour les opérations de rétraction.

On réalise un ensemble d'immobilisation à baïonnette (entre l'outil et la coquille) susceptible de fonctionner dans un sens de rotation comme dans l'autre, donc pour le vissage comme pour de dévissage.

De son côté le noyau 2 destiné à la rétention de la rotule fémorale est fermement ancré dans la coquille 1 par le positionnement des plots 30 dans un des logements 31 ou 32; sa paroi externe convexe formant dôme 2a épouse la partie interne concave de la coquille 1 en lui conférant ainsi, grâce également à la paroi cylindrique 2b emboitée dans la partie cylindrique réceptrice 26, un maintien absolu au sein de l'implant fixe 1.

Le logement 2c interne au noyau 2 destiné à recevoir la rotule fémorale se caractérise par une surface d'appui de diamètre "D" de 28 mm.

On voit à la Figure 4 que ce logement interne du noyau se prolonge au delà du diamètre équatorial "D" pour définir une ouverture d'entrée de diamètre "D1" légèrement inférieur au diamètre équatorial "D".

Cette ouverture est prévue avec un diamètre d'entrée D1 de 27,5mm. assurant ainsi une action de rétention s'exerçant sur une différence de diamètre de 500 microns entre le diamètre équatorial D de la rotule et de son logement récepteur 2a et le diamètre "D1" de la zone d'entrée.

Un léger chanfrein 37 facilite la pénétration de la rotule dans son logement au sein du noyau 2.

L'utilisation d'un noyau de diamètre tel que défini permet compte tenu des différentes tailles de laisser subsister une épaisseur utile de polyéthylène nécessaire pour prévenir les phénomènes d'usure et de déformation (fluage) propres aux résines synthétiques.

L'implant fémoral qui sera décrit ci-après est illustré par les Figures 9, 10 et 11.

La conception de cet implant a retenu dans ces principes directeurs la prise en compte des nécessités suivantes : l'implant scellé doit assurer un remplissage maximum tout en diminuant la quantité de ciment, en accroissant la stabilité primaire et en diminuant les concentrations de contrainte.
La synthèse de ces critères de choix a été rendue possible par l'adoption d'un dessin original notamment au niveau des sections.

La tige de l'implant 4 est constituée d'une pièce en alliage de titane TA6V présentant dans sa vue de face les courbures classiques d'un implant dont l'embase à 45° de l'axe de construction est surmontée d'une collerette en appui à 1 cm. du petit trochanter.

Les sections évoluent selon les variations de la morphologie interne du fémur.

A partir d'expérimentations les inventeurs ont pu déterminer un rapport optimal et critique entre le col antéro-postérieur et le col transversal au niveau du plan de section de la tige au niveau de l'embase. Ce rapport est égal à 0,62 avec tolérance en plus ou moins de 15 %.

La section de l'embase du col sur la tige est en forme de "D" horizontalement oblong. Outre le fait de se conformer à l'anatomie, ceci permet d'éviter l'effet de coin externe et favorise un blocage proximal. La partie trochantérienne tulipée se raccorde à la partie diaphysaire ovoïde par simple lissage des courbes.

L'extrémité inférieure de l'implant est biseautée sur son bord externe pour faciliter l'introduction et diminuer la probabilité de contraintes électives de la pointe sur la corticale externe.

Enfin, le col subit une latéralisation de 0,5 mm. par taille passant de 0 à 2,5 mm.

L'adaptation morphologique d'une prothèse à l'âme du fût Fémoral selon l'invention entraîne :
- l'antéversion physiologique anatomique,
- l'autoblocage par remplissage optimal de la cavité et non par le blocage sur trois points sélectif,
- la suppression de l'effet de coin externe,
- une plus grande facilité d'introduction de l'implant malgré un remplissage plus important,
- une minimisation des contraintes ponctuelles,
- une répartition harmonieuse des charges diffusées sur toute la longueur de l'implant.

## Revendications

1. Prothèse totale de la hanche comportant un implant cotyloïdien constitué d'une coquille hémisphérique (1) comportant sur sa paroi extérieure, dans la partie proche de l'équateur, un filetage hélicoïdal et spiralé laissant dégagée au sommet de la demi-sphère une calotte supérieure (19), le filetage présentant des propriétés d'auto-taraudage permettant ainsi une attaque du milieu d'implantation pour l'insertion et le passage des filets dans le sens de l'implantation de la prothèse, les filets étant interrompus à intervalles réguliers par des gorges (9, 9') disposées selon un axe médian radial (11) et définissant des segments de filets ou ailettes (7, 7', 8, 8') répartis en hélice spiralée sur la paroi de la coquille hémisphérique, au moins dans la zone proche de l'équateur,
caractérisée en ce que le filetage présente des propriétés de rétro-autotaraudage pour une attaque du milieu d'implantation et le passage de filets dans le sens du retrait de la prothèse.

2. Prothèse totale de la hanche selon la revendication 1, caractérisée en ce que les bords de chaque gorge (9, 9') définis par les bords transversaux et terminaux (10, 10') des segments de filets (7, 7', 8, 8') superposés en hélice, sont parallèles à l'axe médian radial (11) de la gorge (9, 9'), lui-même disposé radialement, de sorte que les deux bords (10, 10') de deux segments de filets voisins (7, 8) délimitant entre eux une gorge (9) séparant ces deux segments successifs le long de l'hélice, sont parallèles entre eux et non convergents, et ainsi chaque bord transversal et terminal d'un segment de filet constitue un tranchant d'attaque apte à aborder la matière taraudée selon un angle (α) inférieur à un droit et tel que la matière taraudée reçoit en premier lieu l'attaque de la pointe extérieure dudit bord transversal et terminal du segment de filet pénétrant dans ladite matière taraudée.

3. Prothèse totale de la hanche selon l'une des revendications précédentes, caractérisée en ce que le bord de chaque gorge, défini par le segment d'attaque du filet d'auto-taraudage est parallèle à l'axe médian radial (11) de la gorge (9, 9') n'est pas convergent, et forme ainsi un angle (α) avec le rayon (12) correspondant passant par la pointe extérieure périphérique du filet, de sorte que le filet attaque la matière osseuse par ladite pointe en premier lieu.

4. Prothèse totale de la hanche selon l'une des revendications 1 à 3, caractérisée en ce que chaque gorge (9, 9') comporte une largeur constante, comprise entre 2 et 5 millimètres, et son fond épouse la forme d'un segment de sphère prolongeant la calotte sphérique (19).

5. Prothèse totale de la hanche selon l'une des revendications 1 à 4, caractérisée en ce que les segments de filets ou ailettes (7, 7', 8, 8') définissant ensemble le filetage d'auto-taraudage, sont répartis selon une hélice spiralée de pas compris entre 3 et 4 millimètres et de préférence voisin de 3,5 millimètres.

## Claims

1. Total hip joint prosthesis comprising a cotyloidal implant constituted by a hemispherical shell (1) comprising on its outer wall, in the part close to the equator, a helicoidal and spirally wound threading leaving an upper cap (19) clear at the apex of the demi-sphere, the threading presenting properties of self-tapping thus allowing engagement of the implantation medium for the insertion and passage of the threads in the sense of implantation of the prosthesis, the threads being interrupted at regular intervals by grooves (9, 9') disposed along a radial median axis (11) and defining segments of threads or fins (7, 7', 8, 8') distributed in a spirally wound helix on the wall of the hemispherical shell, at least in the zone close to the equator,
characterized in that the threading presents properties of retro-self-tapping for engagement of the implantation medium and the passage of threads in the sense of withdrawal of the prosthesis.

2. Total hip joint prosthesis according to Claim 1, characterized in that the edges of each groove (9, 9') defined by the transverse and terminal edges (10, 10') of the segments of threads (7, 7', 8, 8') superposed in helical form, are parallel to the radial median axis (11) of the groove (9, 9'), itself disposed radially, with the result that the two edges (10, 10') of two adjacent thread segments (7, 8) together defining a groove (9) separating these two successive segments along the helix, are parallel to each other and not convergent, and each transverse and terminal edge of a segment of thread thus constitutes a cutting edge for engagement adapted to arrive at the tapped matter at an angle (α) less than a right angle and such that the tapped matter firstly receives the engagement of the outer tip of said transverse and terminal edge of the segment of thread penetrating in said tapped matter.

3. Total hip joint prosthesis according to one of the preceding Claims, characterized in that the edge of each groove, defined by the segment of engagement of the self-tapping thread is parallel to the radial median axis (11) of the groove (9, 9') is not convergent, and thus forms an angle (α) with the corresponding radius (12) passing through the peripheral outer tip of the thread, with the result that the thread firstly engages the osseous matter by said tip.

4. Total hip joint prosthesis according to one of Claims 1 to 3, characterized in that each groove (9, 9') presents a constant width, included between 2 and 5 millimeters, and its bottom follows the shape of a segment of sphere extending the spherical cap (19).

5. Total hip joint prosthesis according to one of Claims 1 to 4, characterized in that the segments of threads or fins (7, 7', 8, 8') together defining the self-tapping threading, are distributed in a spirally wound helix whose pitch is included between 3 and 4 millimeters and preferably close to 3.5 millimeters.

## Patentansprüche

1. Hüftgelenkprothese mit einem Hüftpfannenimplantat, bestehend aus einer halbkugelförmigen Schale (1), auf deren Außenwandung in der Nähe der Kugelgürtellinie ein spiralförmiges Schraubengewinde angelegt ist, das an der Spitze der Halbkugel eine obere Kappe (19) freiläßt und selbstschneidende Eigenschaften derart aufweist, daß es zum Einführen und Eindrehen der Gewinde in Richtung der Implantation der Prothese am Implantationsort angreifen kann, wobei das Gewinde in regelmäßigen Abständen von Kehlen (9, 9') unterbrochen ist, die entlang einer radialen Mittelachse (11) angeordnet sind und schrauben- und und spiralförmig über die Wandung der halbkugelförmigen Schale, zumindest im Bereich ihrer Gürtellinie, verteilte Gewindesegmente oder Gewindeflügel (7, 7', 8, 8') ausbilden,
dadurch gekennzeichnet, daß das Gewinde auch in Rückwärtsrichtung selbstschneidende Eigenschaften aufweist, um ein Angreifen am Implantationsort und das Herausdrehen des Gewindes in Entnahmerichtung der Prothese zu ermöglichen.

2. Hüftgelenkprothese nach Anspruch 1, dadurch gekennzeichnet, daß die durch die in Querrichtung verlaufenden Randkanten (10, 10') der schraubenförmig übereinanderliegenden Gewindesegmente (7, 7', 8, 8') gebildeten Ränder der Kehlen (9, 9') sich jeweils parallel zur radialen Mittelachse (11) der selbst ebenfalls radial verlaufenden Kehle (9, 9') erstrecken, so daß die beiden Ränder (10, 10') von zwei benachbarten Gewindesegmenten (7, 8), die eine dazwischenliegende, die beiden Segmente im Bereich des schraubenförmigen Gewindes voneinander trennende Kehle (9) begrenzen, zueinander parallel und nicht konvergierend verlaufen, so daß jede sich in Querrichtung erstreckende und ein Gewindesegment begrenzende Kante eine Schneidkante bildet, die an dem zu schneidenden Material unter einem Winkel (α) angreift, der kleiner ist als ein rechter Winkel, so daß zuerst die äußere Spitze der in Querrichtung verlaufenden Endkante des Gewindesegments an dem zu schneidenden Material angreift und in dieses eindringt.

3. Hüftgelenkprothese nach einem der vorhergehenden Ansprüche, dadurch gekennzeichnet, daß die durch das angreifende Segment des selbstschneidenden Gewindes gebildete Kante jeder Kehle parallel zur radialen Mittelachse (11) der Kehle (9, 9') und nicht konvergierend verläuft und auf diese Weise mit dem entsprechenden, durch die äußere periphere Spitze des Gewindes hindurchgehenden Radius (12) einen Winkel (α) bildet, so daß das Gewinde zuerst mit dieser Spitze am Knochenmaterial angreift.

4. Hüftgelenkprothese nach einem der Ansprüche 1 bis 3, dadurch gekennzeichnet, daß jede Kehle (9, 9') eine konstante Breite zwischen 2 und 5 mm aufweist und der Boden der Kehle sich der Form eines die Verlängerung der kugelförmigen Kappe (19) bildenden Kugelsegments anpaßt.

5. Hüftgelenkprothese nach einem der Ansprüche 1 bis 4, dadurch gekennzeichnet, daß die zusammen das selbstschneidende Gewinde bildenden Gewindesegmente oder Gewindeflügel (7, 7', 8, 8') schrauben- und spiralförmig mit einer Steigung zwischen 3 und 4 mm, vorzugsweise im Bereiche von 3,5 mm, verteilt sind.
